# EUROPEAN PATENT APPLICATION

(11) **EP 4 810 017 A1**
(43) Date of publication of application: **23.09.2026**
(21) Application number: 25210507.7
(22) Date of filing: 22.10.2025
(51) Int. Cl.: A61K 9/00, A61K 9/20, A61K 31/165, A61P 25/24

(54) **A CHEWABLE TABLET FORMULATION OF LISDEXAMFETAMINE DIMESYLATE**

(30) Priority: 20.03.2025 TR 2025003338
(71) Applicant: Sanovel Ilac Sanayi Ve Ticaret Anonim Sirketi, Istanbul (TR)
(72) Inventor: SUNEL, Fatih, Istanbul (TR); ATAK, Faidme Bilgehan, Istanbul (TR); ATAMAN, Seval, Istanbul (TR); YUCEL, Alpay, Istanbul (TR)
(74) Representative: Genç Ilhan, Oznur

(57) **Abstract**

The present invention relates to a chewable tablet formulation comprising lisdexamfetamine dimesylate and at least one filler, wherein the amount of the lisdexamfetamine dimesylate is between 0.1% and 10.0% by weight in the total formulation. Furthermore, the invention discloses a process which is simple, rapid, cost effective, time-saving and industrially convenient process.

## Description

### Field of the Invention

The present invention relates to a chewable tablet formulation comprising lisdexamfetamine dimesylate and at least one filler, wherein the amount of the lisdexamfetamine dimesylate is between 0.1% and 10.0% by weight in the total formulation. Furthermore, the invention discloses a process which is simple, rapid, cost effective, time-saving and industrially convenient process.

### Background of the Invention

Lisdexamfetamine dimesylate is used to treat attention deficit hyperactivity disorder (ADHD) in adults and children 6 years of age and older. This medicine is also used to treat moderate to severe binge eating disorder (BED). It belongs to the group of medicines called central nervous system (CNS) stimulants.

Lisdexamfetamine dimesylate increases attention and decreases restlessness in children and adults who are overactive, cannot concentrate for very long, or are easily distracted and impulsive. This medicine is used as part of a total treatment program that also includes social, educational, and psychological treatment.

Lisdexamfetamine dimesylate also referred to as I-lysine-d-amphetamine dimesylate of Formula I is chemically named as (2S)-2,6-Diamino-N-[(1S)-1-methyl-2-phenylethyl]hexanamide dimesylate.

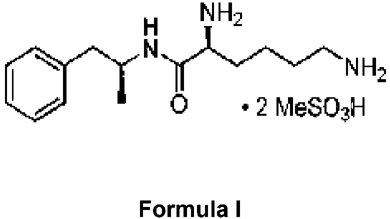

Lisdexamfetamine dimesylate is a white to off-white powder, very soluble in water and soluble in methanol, melting point 192-198°C, molecular formula C₁₇H₃₃N₃O₇S₂ and molecular weight 455.59 g/mol.

Lisdexamfetamine is currently commercially available only as 10, 20, 30, 40, 50 and 60 mg hard capsules or chewable tablets. Each capsule or chewable tablet contains 10, 20, 30, 40, 50 or 60 mg lisdexamfetamine dimesylate, equivalent to 5.8 mg, 11.6 mg, 17.3 mg, 23.1 mg, 28.9 mg or 34.7 mg lisdexamfetamine, respectively.

The patent WO2006121552A2 discloses a capsule and chewable tablet formulation containing lisdexamfetamine dimesylate, microcrystalline cellulose, croscarmellose sodium, magnesium stearate.

There are examples of lisdexamfetamine dimesylate in chewable tablet form in the prior art, but it is not sufficient. There are a number of challenges, that are not sufficiently detailed in the prior art. There is still a need for a formulation that improves flowability, compressibility and content uniformity while ensuring ease of use and patient compliance with the product.

### Detailed Description of the Invention

The main objective of the present invention is to provide a chewable tablet formulation comprising lisdexamfetamine dimesylate that has patient compliance and ease of use of the product and improves its flowability, compressibility and content uniformity.

Another object of the present invention is to provide a chewable tablet formulation comprising lisdexamfetamine dimesylate having high stability.

Another object of the present invention is to provide a process for a chewable tablet formulation comprising lisdexamfetamine dimesylate prepared by simple, easy, time-saving and fast manufacturing methods.

Lisdexamfetamine dimesylate is a deliquescent active substance. Deliquescent active ingredients tend to absorb moisture from the atmosphere and liquefy. These substances should be used carefully, especially in some pharmaceutical formulations. Since deliquescent active substances are prone to absorb humidity and liquefy, the excipients to be used and manufacturing method should be decided carefully. That may cause flowability, compression, and content uniformity problems in tablet and capsule dosage forms. For this reason, a formulation study was carried out that is good for flowability, compression and content uniformity, as well as patient compliance and ease of use of the product. Surprisingly, it was found that the use of lisdexamfetamine dimesylate between 0.1% and 10.0% and using at least one filler overcame all of these problems.

According to one embodiment of the invention, a chewable tablet formulation comprising lisdexamfetamine dimesylate and at least one filler, wherein the amount of the lisdexamfetamine dimesylate is between 0.1% and 10.0% by weight in the total formulation. The ratio of active substance amount to the total tablet weight in the form enables an ideal volume for the tablet that results in ease of use of the product and patient compliance.

The active substance used in low doses causes some problems such as homogeneity and content uniformity. In the present invention, in order to overcome this problem, the following excipients are preferred with the priority of compatibility with lisdexamfetamine dimesylate. In particular, the use of appropriate filler helps to ensure the physicochemical compatibility with lisdexamfetamine dimesylate and improved powder flow, compressibility and content uniformity in manufacturing.

Suitable fillers are selected from the group comprising microcrystalline cellulose, D-mannitol, isomalt, sorbitol, lactose, lactose monohydrate, mannitol, spray-dried mannitol, dextrose, sucrose, fructose, maltose, inorganic salts, calcium salts, polysaccharides, dicalcium phosphate, sodium chloride, dextrates, lactitol, sucrose-maltodextrin mixture, trehalose, sodium carbonate, sodium bicarbonate, calcium carbonate or mixtures thereof.

According to this embodiment of the present invention, the filler is microcrystalline cellulose or D-mannitol or isomalt or sorbitol or mixtures thereof.

When using fillers selected above, both flowability and compressibility problems are eliminated. Good flowability and compressibility ensure homogeneous distribution of the active ingredients and uniform pressing of the tablets. This improves content uniformity.

In addition, the moisture content of microcrystalline cellulose used was below 1.5%. In this way, it helped to decrease moisture content of chewable tablets and increase the stability of lisdexamfetamine dimesylate, which is sensitive to moisture.

According to this embodiment of the present invention, the amount of filler is between 55.0% and 95.0% by weight in the total formulation. Preferably, the amount of filler is between 65.0% and 90.0% by weight in the total formulation.

According to an embodiment of the present invention, the chewable tablet formulation further comprises at least one pharmaceutically acceptable excipient which is selected from the group comprising binders, lubricants, sweeteners, flavouring agents, stabilizers, disintegrants, glidants or mixtures thereof.

Suitable binders are selected from the group comprising polyvinylpyrrolidone, pregelatinized starch, maltodextrin, carboxymethylcellulose sodium, sugars, agar, alginates, carbomers, cellulose acetate phthalate, chitosan, starch mucilage, acacia mucilage, dextrates, dextrin, dextrose, ethylcellulose, glyceryl behenate, hydrogenated vegetable oil type I, hydroxyethyl cellulose, hydroxyethylmethyl cellulose, hydroxypropyl cellulose, hydroxypropyl starch, hypromellose, magnesium aluminum silicate, methylcellulose, poloxamer, polycarbophil, polydextrose, polyethylene oxide, polymethacrylates, aluminia hydroxide, stearic acid, sucrose, polyoxyethylene-alkyl ethers, pullulan or mixtures thereof.

According to this embodiment of the present invention, the binder is polyvinylpyrrolidone or pregelatinized starch or maltodextrin or mixtures thereof.

According to this embodiment of the present invention, the amount of binder is between 1.0% and 15.0% by weight in the total formulation.

Suitable lubricants are selected from the group comprising sodium stearyl fumarate, magnesium stearate, talc, calcium stearate, zinc stearate, magnesium lauryl sulfate, sodium oleate, sodium acetate, stearic acid, fumaric acid, or mixtures thereof.

According to one embodiment of the present invention, the lubricant is sodium stearyl fumarate or magnesium stearate or talc or mixture thereof.

According to one embodiment of the present invention, the amount of lubricant is between 0.01% and 8.0% by weight in the total formulation.

Suitable sweeteners are selected from the group comprising xylitol, sucralose, aspartame, sorbitol, erythritol, thaumatin, mogroside, inulin, acesulfame-K, saccharin or its sodium and calcium salts, sodium cyclamate, sucrose, glucose, menthol, peppermint, cinnamon or mixtures thereof.

According to an embodiment of the present invention, the sweeteners is xylitol or sucralose or mixtures thereof.

According to this embodiment of the present invention, the amount of sweetener is between 0.01% and 20.0% by weight in the total formulation.

Suitable flavouring agents are selected from the group comprising lemon cream, peppermint, menthol, orange, cherry, cinnamon, chocolate, vanillin, strawberry, grape, black currant, raspberry, banana, red fruits, wild berries or mixtures thereof.

According to this embodiment of the present invention, the amount of flavouring agent is between 0.001% and 5.0% by weight in the total formulation.

According to an embodiment of the present invention, the flavouring agent is lemon cream, peppermint or raspberry or mixtures thereof.

Suitable stabilizers are selected from the group comprising magnesium aluminometasilicate, glycerol dibehenate, citric acid, fumaric acid, tartaric acid, sodium citrate, sodium benzoate, sodium dihydrogen phosphate, calcium carbonate, magnesium carbonate, arginine, lysine, meglumine, ascorbic acid, gallic acid esters or the mixtures thereof.

According to an embodiment of the present invention, the stabilizer is magnesium aluminometasilicate. When these were used, thus obtaining a formulation with high stability.

According to one embodiment of the present invention, the amount of the stabilizer is between 1.0% and 20.0% by weight of the total formulation. Preferably, it is between 5.0% and 15.0% by weight in the total formulation.

Suitable disintegrants are selected from the group comprising crospovidone, croscarmellose sodium, sodium starch glycolate, low-substituted hydroxypropyl cellulose, starch, calcium carboxymethyl cellulose, pregelatinized starch, docusate sodium, guar gum, sodium alginate, alginic acid, magnesium aluminum silica, poloxamer, sodium glycine carbonate or mixtures thereof.

According to one embodiment of the present invention, the disintegrant is crospovidone or croscarmellose sodium or mixture thereof.

According to one embodiment of the present invention, the amount of disintegrant is between 0.01% and 10.0% by weight in the total formulation.

Suitable glidants are selected from the group comprising colloidal silicon dioxide, aluminum silicate, starch or mixtures thereof.

According to one embodiment of the present invention, the glidant is colloidal silicon dioxide.

Processes for preparing a chewable tablet comprising a micronized form of an active ingredient, the method comprising the steps of combining the active ingredient with tablet excipients by geometric dilution to form a final mixture and applying direct compression to at least a portion of the final mixture to form at least one tablet.

Our chewable tablet formulation, prepared by the geometric mixing method, provides high homogeneity and effectiveness by ideally combining the micronized form the active ingredient and tablet excipients, and provides a successful tableting process with the use of special technical devices that overcome difficulties such as poor flowability. This approach increases homogeneous dispersion and therefore content uniformity.

According to this embodiment of the invention, a process for chewable tablet formulations comprising lisdexamfetamine dimesylate comprises the following steps:
- Sieving and geometric mixing all components with blender and pressing with compression force.

### Example 1;

| **Ingredients** | **%by weight** | **%by weight** |
|---|---|---|
| Lisdexamfetamine Dimesylate | 2.9 | 0.1-10 |
| Pregelatinized starch | 2.9 | 0.1-10 |
| Maltodextrin | 8.6 | 1-15 |
| Croscarmellose Sodium | 2.9 | 0.1-10 |
| Sorbitol | 35.1 | 25-45 |
| Microcrystalline Cellulose | 46.6 | 35-55 |
| Sucralose | 0.1 | 0.01-5 |
| Sodium Stearyl Fumarate | 0.1 | 0.01-5 |
| Lemon cream | 0.4 | 0.01-5 |
| Peppermint | 0.4 | 0.01-5 |
| **TOTAL** | **100** | 100 |

- Sieving and geometric mixing all components with blender and pressing with compression force.

### Example 2;

| **Ingredients** | **%by weight** | **%by weight** | **%by weight** |
|---|---|---|---|
| Lisdexamfetamine Dimesylate | 2.9 | 3.3 | 0.1-10 |
| D- mannitol | 35.7 | 33.3 | 25-45 |
| Xylitol | 10 | 11.7 | 1-20 |
| Microcrystalline Cellulose | 38.4 | 36.5 | 25-45 |
| Crospovidone | 4.3 | 5 | 0.5-10 |
| Magnesium Aluminometasilicate | 8.6 | 10 | 1-15 |
| Magnesium stearate | 0.1 | 0.2 | 0.01-5 |
| Raspberry flavour | 0.01 | 0.02 | 0.001-5 |
| **TOTAL** | **100** | **100** | **100** |

- Sieving and geometric mixing all components with blender and pressing with compression force.

### Example 3;

| **Ingredients** | **%by weight** | **%by weight** |
|---|---|---|
| Lisdexamfetamine Dimesylate | 3.3 | 0.5-10 |
| Isomalt | 50 | 40-60 |
| Povidone | 4 | 0.5-10 |
| Microcrystalline Cellulose | 34.4 | 25-45 |
| Croscarmellose Sodium | 4.3 | 0.5-10 |
| Talc | 3.3 | 0.5-10 |
| Sucralose | 0.2 | 0.01-5 |
| Colloidal Silicon dioxide | 0.2 | 0.01-5 |
| Magnesium stearate | 0.2 | 0.01-5 |
| Artificial Strawberry Flavor | 0.07 | 0.001-5 |
| **TOTAL** | **100** | |

- Sieving and geometric mixing all components with blender and pressing with compression force.

## Claims

1. A chewable tablet formulation comprising lisdexamfetamine dimesylate and at least one filler, wherein the amount of the lisdexamfetamine dimesylate is between 0.1% and 10.0% by weight in the total formulation.

2. The chewable tablet formulation according to claim 1, wherein fillers are selected from the group comprising lactose monohydrate, microcrystalline cellulose, D-mannitol, isomalt, sorbitol, lactose, mannitol, spray-dried mannitol, dextrose, sucrose, fructose, maltose, inorganic salts, calcium salts, polysaccharides, dicalcium phosphate, sodium chloride, dextrates, lactitol, sucrose-maltodextrin mixture, trehalose, sodium carbonate, sodium bicarbonate, calcium carbonate or mixtures thereof.

3. The chewable tablet formulation according to claim 1 or 2, wherein the filler is microcrystalline cellulose or D-mannitol or isomalt or sorbitol or mixtures thereof.

4. The chewable tablet formulation according to claim 1, wherein the chewable tablet formulation further comprises at least one pharmaceutically acceptable excipient which is selected from the group comprising binders, lubricants, sweeteners, flavouring agents, stabilizers, disintegrants, glidants or mixtures thereof.

5. The chewable tablet formulation according to claim 4, wherein binders are selected from the group comprising polyvinylpyrrolidone, pregelatinized starch, maltodextrin, carboxymethylcellulose sodium, sugars, agar, alginates, carbomers, cellulose acetate phthalate, chitosan, starch mucilage, acacia mucilage, dextrates, dextrin, dextrose, ethylcellulose, glyceryl behenate, hydrogenated vegetable oil type I, hydroxyethyl cellulose, hydroxyethylmethyl cellulose, hydroxypropyl cellulose, hydroxypropyl starch, hypromellose, magnesium aluminum silicate, methylcellulose, poloxamer, polycarbophil, polydextrose, polyethylene oxide, polymethacrylates, aluminia hydroxide, stearic acid, sucrose, polyoxyethilene-alkyl ethers, pullulan or mixtures thereof.

6. The chewable tablet formulation according to claim 4 or 5, wherein the binder is polyvinylpyrrolidone or pregelatinized starch or maltodextrin or mixtures thereof.

7. The chewable tablet formulation according to claim 4, wherein lubricants are selected from the group comprising sodium stearyl fumarate, magnesium stearate, talc, calcium stearate, zinc stearate, magnesium lauryl sulfate, sodium oleate, sodium acetate, stearic acid, fumaric acid, or mixtures thereof.

8. The chewable tablet formulation according to claim 4 or 7, wherein the lubricant is sodium stearyl fumarate or magnesium stearate or talc or mixture thereof.

9. The chewable tablet formulation according to claim 4, wherein sweeteners are selected from the group comprising xylitol, sucralose, aspartame, sorbitol, erythritol, thaumatin, mogroside, inulin, acesulfame-K, saccharin or its sodium and calcium salts, sodium cyclamate, sucrose, glucose, menthol, peppermint, cinnamon or mixtures thereof.

10. The chewable tablet formulation according to claim 4 or 9, wherein the sweetener is xylitol or sucralose or mixtures thereof.

11. The chewable tablet formulation according to claim 4, wherein flavouring agents are selected from the group comprising lemon cream, peppermint, menthol, orange, cherry, cinnamon, chocolate, vanillin, strawberry, grape, black currant, raspberry, banana, red fruits, wild berries or mixtures thereof.

12. The chewable tablet formulation according to claim 4, wherein stabilizers are selected from the group comprising magnesium aluminometasilicate, glycerol dibehenate, citric acid, fumaric acid, tartaric acid, sodium citrate, sodium benzoate, sodium dihydrogen phosphate, calcium carbonate, magnesium carbonate, arginine, lysine, meglumine, ascorbic acid, gallic acid esters or the mixtures thereof.

13. The chewable tablet formulation according to claim 4 or 12, wherein the stabilizer is magnesium aluminometasilicate.

14. The chewable tablet formulation according to claim 4, wherein disintegrants are selected from the group comprising crospovidone, croscarmellose sodium, sodium starch glycolate, low-substituted hydroxypropyl cellulose, starch, calcium carboxymethyl cellulose, pregelatinized starch, docusate sodium, guar gum, sodium alginate, alginic acid, magnesium aluminum silica, poloxamer, sodium glycine carbonate or mixtures thereof.

15. The chewable tablet formulation according to claim 4 or 14, wherein the disintegrant is crospovidone or croscarmellose sodium or mixture thereof.
